# EUROPEAN PATENT SPECIFICATION

(11) **EP 4 251 256 B1**
(45) Date of publication and mention of the grant of the patent: **17.09.2025**
(21) Application number: 21819660.8
(22) Date of filing: 09.11.2021
(51) Int. Cl.: A61M 39/08

(54) **MEDICAL DEVICES FOR ANTI-BUBBLE MEDICATION DELIVERY**
MEDIZINISCHE VORRICHTUNGEN ZUR BLASENFREIEN MEDIKAMENTENVERABREICHUNG
DISPOSITIFS MÉDICAUX POUR ADMINISTRATION ANTIBULLE DE MÉDICATION

(30) Priority: 24.11.2020 US 202017103459
(43) Date of publication of application: 04.10.2023
(73) Proprietor: CareFusion 303, Inc., San Diego, CA 92130 (US)
(72) Inventor: TRAINER, Lawrence J., Murray, Utah 84107 (US); SEVINC, Zehra, Round Lake, Illinois 60073 (US); BOGGAVARAPU, Sajiv, Vernon Hills, Illinois 60061 (US)
(74) Representative: Zenz Patentanwälte Partnerschaft mbB
(86) International application number: PCT/US2021/058657
(87) International publication number: WO 2022/115235

(56) References cited:
- EP-A2- 2 792 380
- US-A1- 2014 123 981
- US-A1- 2018 369 556

## Description

### CROSS-REFERENCES TO RELATED APPLICATIONS

This Application claims the benefit of U.S. Patent Application No. 17/103,459 entitled "MEDICAL DEVICES FOR ANTI-BUBBLE MEDICATION DELIVERY," filed on November 24, 2020.

### BACKGROUND

Infusion of medication is often done by infusion pump systems or gravity fed systems and corresponding intravenous (IV) sets. Typical IV sets have infusion components such as drip chambers, check valves, roller clamps, filters and tubing couplers, all connected together by primary and secondary IV tubing. The infusion components and tubing must be primed by placing IV fluid in the infusion components and IV tubing to remove all air prior to attaching the IV tube to the patient. IV tubing is primed to prevent air from entering the circulatory system, thus preventing an air embolism, which is a potential complication of IV therapy. Air embolisms can enter a patient's blood system through cut or unprimed IV tubing, infusion component access ports, and infusion components with too little fluid. Air embolisms can be fatal and deaths have occurred with as little as 10 ml of air. Known solutions to minimize or prevent air bubbles from occurring include using adaptors, sensors and membrane technologies to trap air bubbles in order to minimize bubbles and speed-up priming. However, these solutions require additional devices that increase the complexity of the IV set, while resulting in the need for training users on the correct use of the air bubble removing devices.

It is desirable to provide infusion components and IV tubing having hydrophilic material to prevent air bubbles from sticking to fluid path surfaces, thereby reducing the occurrence of air embolisms, reducing priming time, eliminating the need for additional training and improving manufacturing costs by eliminating the need for additional devices.

US 2018/369556 A discloses a medical instrument including a first tube body and a second tube body joined to the first tube body by being inserted in the lumen of the first tube body. The second tube body is more rigid than the first tube body. The medical instrument includes a coating layer made of a biocompatible material disposed on the inner peripheral surfaces of the first and second tube bodies. The inner peripheral surface of the second tube body is radially inward of the inner peripheral surface of the first tube body to create a level difference portion. The thickness of the part of the coating layer which coats the level difference portion is larger than the thickness of the level difference portion.

EP 2 792 380 A2 discloses the use of acrylic acid or acrylic acid anhydride for hydrophilizing surfaces of a body comprises pre-coating the surfaces using a high frequency plasma generated from a gas mixture composed of an inert gas and a first gas formed of biocompatible, polymerizable, carboxy group-containing monomers, and then follow-up coating the precoated surfaces of the body, wherein the pre-coating takes less than 10 minutes or even less than 1 minute, and the follow-up coating is made using a second gas substantially consisting of the acrylic acid or acrylic acid anhydride monomers.

US 2014/123981 Al discloses skin-contact products with a transpiration function such as medical devices or medicinal products, of which face masks, aspirators, ventilators, breast pumps or wound dressings are examples are described especially a skin-contact product with a transpiration function with an improved microclimate at a patient interface material-skin contact area. In an embodiment a material system is described that comprises a hydrophobic silicone base material and a hydrophilic silicone material that is combined with the hydrophobic base material. At least a part of the hydrophilic material is in contact with a moist surface. The hydrophobic base material provides mechanical and dynamical stability of the material system. The hydrophilic material allows for uptake or for diffusion of moisture away from moist surface. The material system is utilized to fabricate a patient interface material and forehead pad of a user or patient interface, such as face mask, for example, a patient interface mask for positive air pressure therapy. Furthermore, a novel composition for the preparation of hydrophilic silicone materials, suitable for use in the material system, is disclosed.

### SUMMARY

The present disclosure provides medical tubing and infusion components having hydrophilic coatings and/or hydrophilic blended base materials, decreasing the water wetting angle to cause air bubbles to slide away quickly or pop off and disappear after contacting the hydrophilic surface, thus reducing both the time required for priming and the risk of an air embolism from fluid delivery of an IV set.

In one or more embodiments, a hydrophilic infusion tube is provided. The hydrophilic infusion tube includes a flexible tube configured to connect a first fluid port and a second fluid port, the flexible tube comprising a base material suitable for fluid infusion and a hydrophilic fluid interfacing surface disposed on an inner surface of the flexible tube wherein the hydrophilic fluid interfacing surface provides a water wetting contact angle that is less than a water wetting contact angle provided by the base material, wherein the hydrophilic fluid interfacing surface comprises a blend of the base material with a hydrophilic additive during formation of the flexible tube.

In one or more aspects, the base material includes one or more of thermoplastic polyurethane (TPU), thermoplastic elastomer (TPE), thermoplastic polyolefin (TPO), soft polyvinyl chloride (PVC), silicone, thermoplastic vulcanizate (TPV) (ethylene propylene diene monomer (EPDM) + polypropylene (PP)), thermoplastic styrenic elastomer (TPS) (styrene-butadiene-styrene (SBS) /styrene-ethylene-butylene-styrene (SEBS) /styrene-isoprene-rubber (SIS) /styrene-ethylene-propylene-styrene (SEPS)) and its blending with polyolefin, and thermoplastic polyester elastomer (TPEE) (polyether ester) rubber). In one or more aspects, the hydrophilic fluid interfacing surface comprises a hydrophilic material coated on the base material. In one or more aspects, the hydrophilic material includes Guanidinium copolymer. In one or more aspects, the hydrophilic material provides a lower coefficient of friction than a coefficient of friction provided by the base material. In one or more aspects, the hydrophilic material provides a material wettability that is higher than a material wettability provided by the base material.

In one or more aspects, the hydrophilic fluid interfacing surface comprises a hydrophilic material grafted on the base material. In one or more aspects, the hydrophilic fluid interfacing surface comprises a blend of the base material with a hydrophilic additive. In one or more aspects, the hydrophilic additive includes one or more of polyvinyl pyrrolidone (PVP), polyvinyl alcohol, chitosan (CS), polyamide, polyethylene oxide (PEO), polyethylene glycol (PEG), and polyetheramine. In one or more aspects, the hydrophilic additive includes Guanidinium copolymer. In one or more aspects, the hydrophilic additive is up to 10 percent of the weight of the base material and hydrophilic additive combined. In one or more aspects, the hydrophilic additive is up to 20 percent of the weight of the base material and hydrophilic additive combined.

In one or more embodiments, a hydrophilic infusion component is provided. The hydrophilic infusion component includes a housing. The housing includes a first fluid port, a second fluid port, a base material suitable for fluid infusion and a fluid pathway disposed between the first fluid port and the second fluid port. A hydrophilic fluid interfacing surface is disposed on an inner surface of the fluid pathway. The hydrophilic fluid interfacing surface provides a water wetting contact angle that is less than a water wetting contact angle provided by the base material.

In one or more aspects, the base material includes one or more of thermoplastic polyurethane (TPU), thermoplastic elastomer (TPE), thermoplastic polyolefin (TPO), soft polyvinyl chloride (PVC), silicone, thermoplastic vulcanizate (TPV) (ethylene propylene diene monomer (EPDM) + polypropylene (PP)), thermoplastic styrenic elastomer (TPS) (styrene-butadiene-styrene (SBS) /styrene-ethylene-butylene-styrene (SEBS) /styrene-isoprene-rubber (SIS) /styrene-ethylene-propylene-styrene (SEPS)) and its blending with polyolefin, and thermoplastic polyester elastomer (TPEE) (polyether ester) rubber). In one or more aspects, the hydrophilic fluid interfacing surface includes a hydrophilic material coated on the base material. In one or more aspects, the hydrophilic fluid interfacing surface includes a hydrophilic material grafted on the base material. In one or more aspects, the hydrophilic fluid interfacing surface includes a blend of the base material with a hydrophilic additive. In one or more aspects, the hydrophilic additive includes one or more of polyvinyl pyrrolidone (PVP), polyvinyl alcohol, chitosan (CS), polyamide, polyethylene oxide (PEO), polyethylene glycol (PEG), polyetheramine, and Guanidinium copolymer.

In one or more embodiments, an infusion set is provided. The infusion set includes an infusion component and a hydrophilic infusion tube. The hydrophilic infusion tube includes a flexible tube configured to connect a fluid port of the infusion component to a different fluid port, the flexible tube comprising a base material suitable for fluid infusion. The hydrophilic infusion also tube includes a hydrophilic fluid interfacing surface disposed on an inner surface of the flexible tube. The hydrophilic fluid interfacing surface provides a water wetting contact angle that is less than a water wetting contact angle provided by the base material. The flexible tube is configured to provide fluid passage without an accumulation of air bubbles on the inner surface of the flexible tube.

In one or more aspects, the infusion component is a hydrophilic infusion component that includes a housing that includes a first fluid port, a second fluid port, a base material suitable for fluid infusion and a fluid pathway disposed between the first fluid port and the second fluid port, wherein a hydrophilic fluid interfacing surface is disposed on an inner surface of the fluid pathway, and wherein the hydrophilic fluid interfacing surface provides a water wetting contact angle that is less than a water wetting contact angle provided by the base material.

Additional features and advantages of the disclosure will be set forth in the description below and, in part, will be apparent from the description or may be learned by practice of the disclosure. The objectives and other advantages of the disclosure will be realized and attained by the structure particularly pointed out in the written description and claims hereof as well as the appended drawings.

It is to be understood that both the foregoing general description and the following detailed description are exemplary and explanatory and are intended to provide further explanation of the disclosure as claimed.

### BRIEF DESCRIPTION OF THE DRAWINGS

The accompanying drawings, which are included to provide further understanding of the disclosure and are incorporated in and constitute a part of this specification, illustrate embodiments of the disclosure and together with the description serve to explain the principles of the disclosure.
FIG. 1 depicts a schematic view of a typical assembled infusion set.
FIG. 2 depicts a schematic view of effects of surface coating on water wetting angles.
FIG. 3 is a perspective view of a control slide during an air bubble test, according to some aspects of the disclosure.
FIG. 4 is a perspective view of a hydrophilic coated slide during an air bubble test, according to some aspects of the disclosure.
FIG. 5 is a perspective view of a hydrophobic coated slide during an air bubble test, according to some aspects of the disclosure.
FIG. 6 is a perspective view of a super hydrophobic coated slide during an air bubble test, according to some aspects of the disclosure.
FIG. 7 is a perspective view of a control plaque during an air bubble test, according to some aspects of the disclosure.
FIG. 8 is a perspective view of a hydrophilic coated plaque during an air bubble test, according to some aspects of the disclosure.
FIG. 9 is a perspective view of the hydrophilic coated plaque of FIG. 7 incrementally later in time during the air bubble test, according to some aspects of the disclosure.
FIG. 10 is a cross-section front view of a tube and infusion component junction with hydrophilic coating, according to some aspects of the disclosure.
FIG. 11 is a front view of a junction of a tube and infusion component junction with hydrophilic material, according to some aspects of the disclosure.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions are provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

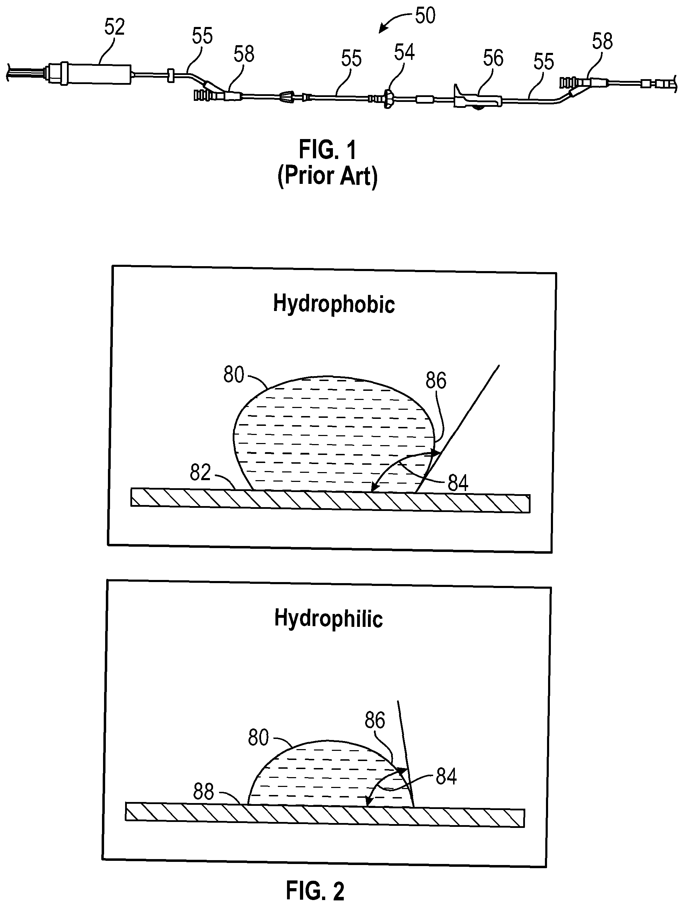

As shown in FIG. 1, a typical infusion set 30 has several different components, including a drip chamber 40, a check valve 50, a roller clamp 60 and Y-junctions 70, all connected together by tubing 20. A typical infusion set 30 can include additional infusion components (e.g., pinch clamps, filters) and can be formed of any combination of components and the tubing 20. The addition of adaptors, sensors and membrane technologies have been used to trap air bubbles in order to minimize bubbles and speed-up priming.

FIG. 2 illustrates effects of surface coating on water wetting angles (e.g., contact angle). For example, the top portion of FIG. 2 illustrates a water droplet 80 on a hydrophobic coated film 82. As shown, the water droplet 80 pulls away from the hydrophobic coated film 82 into a cut off oval shape, providing a contact angle 84 greater than 90 degrees between an outer surface 86 of the water droplet 80 and the hydrophobic coated film 82. By contrast, as shown in the bottom portion of FIG. 2, a water droplet 80 on a hydrophilic coated film 88 does not pull away from the hydrophilic coated film 88 and thus has a semi-spherical shape, providing a contact angle 84 less than 90 degrees between an outer surface 86 of the water droplet 80 and the hydrophilic coated film 88.

According to aspects of the disclosure, it is desirable to provide IV sets and IV components having hydrophilic properties that resist stickiness of air bubbles to the fluid interfacing surfaces. In one specific example, a clear need exists for hazardous drug safety products to prevent hazardous drug exposure to health care workers using secondary lines to administer chemotherapy, where typical air mitigation solutions require increased priming time or additional handling of adaptors, sensors or membrane technologies. By using the subject technology having hydrophilic properties, the hazardous drug exposure is minimized, thereby reducing significant health risks (e.g., safety), increasing regulatory and guideline compliance (e.g., regulations) and reducing time required to prime a secondary line filled with hazardous drugs (e.g., workflow).

According to aspects of the disclosure, the subject technology renders hydrophilic surface properties to the materials used for fluid interfacing surfaces of infusion components and IV tubing. These hydrophilic surfaces provide superior lubricity over hydrophobic surfaces, and the hydrophilic surfaces increase the wettability of the material, resulting in that an air bubble will not stick to the surface (e.g., tube wall). Also, a lower coefficient of friction due to the hydrophilic surface may allow air to slide across the surface more quickly. Thus, these hydrophilic surface properties have a bubble terminating feature that causes air bubbles to slide away quickly while disappearing from the hydrophilic surface, preventing sticking of air bubbles to the base material (e.g., fluid interfacing surfaces) of infusion components and IV tubing, thereby improving fluid flow and providing bubble free priming. These hydrophilic material based infusion components and tubing provide improved ease of use to users and eliminates the need for additional user training. In addition, the hydrophilicity of the fluid interfacing surfaces may provide anti-fouling properties to the infusion components and IV tubing, which may provide for longer use of the infusion components/IV tubing/IV sets before needing to be replaced, as well as improve the effectiveness of the fluid delivery.

According to aspects of the disclosure, the subject technology may be used in any medication delivery system that uses plastic components. The subject technology may be utilized at any component of the medication delivery system having a high risk of air bubble formation and/or at critical components. For example, tubing is one place air typically sticks, while a drip chamber is another area where air bubbles may be created during priming and flow regulation or initial flow rate setting steps.

In some aspects of the disclosure, a hydrophilic surface may be provided by coating the desired surfaces of a tube 20 or infusion component, such as a drip chamber 40, for example. Here, hydrophilic chemicals may be coated onto the desired base material. The base material may include any suitable material, such as thermoplastic polyurethane (TPU), thermoplastic elastomer (TPE), thermoplastic polyolefin (TPO), soft polyvinyl chloride (PVC), silicone, thermoplastic vulcanizate (TPV) (ethylene propylene diene monomer (EPDM) + polypropylene (PP)), thermoplastic styrenic elastomer (TPS) (styrene-butadiene-styrene (SBS) /styrene-ethylene-butylene-styrene (SEBS) /styrene-isoprene-rubber (SIS) /styrene-ethylene-propylene-styrene (SEPS)) and its blending with polyolefin, thermoplastic polyester elastomer (TPEE) (polyether ester) rubber), and the like. A hydrophilic property of a surface may be provided by coating techniques and/or customized chemistry and synthesis through grafting approaches.

Variables of hydrophilic coating may be selected or controlled to achieve the desired hydrophilic properties. For example, such variables may include the type and chemistry of the coating technique, coating thickness, potential interactions with processing or coating application techniques, and geometry limiting the application process.

In some aspects of the disclosure, a hydrophilic surface may be provided by blending hydrophilic chemicals or additives with the desired base material. The base material may include any suitable material, such as a flexible non-PVC (e.g., s-TPE/TPO/TPU or PVC material blends with hydrophilic additives). For example, polyvinyl pyrrolidone (PVP), polyvinyl alcohol, chitosan (CS), polyamide, polyethylene oxide (PEO), and polyethylene glycol (PEG) may provide effective reduction of contact angles 84 when added up to 10wt%. Commercially available hydrophilic additives such as Irgasurf HL560 for PP and Elastamine^{®} polyetheramines for polyolefin base materials may be used. Guanidinium copolymer is another hydrophilic chemical that may be either blended or coated. Additives of hydrophilic nature may be blended up to 20wt% within base polymers to provide different efficiencies.

Variables of hydrophilic blending may be selected or controlled to achieve the desired hydrophilic properties. For example, such variables may include the percentage of the additive(s), types of additives, base material chemistry, processing techniques and the like.

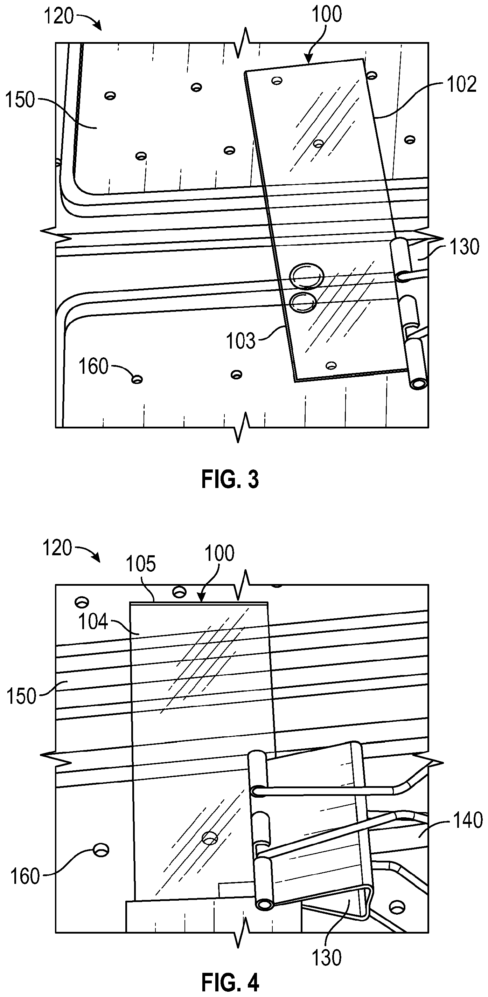

FIGS. 3-9 illustrate control samples (e.g., uncoated) and experimental samples (e.g., coated) of a slide or plaque shaped standard polycarbonate material 100. The samples were tested in a test apparatus 120 and test procedure to compare the air response on various surfaces of the sample plastic plaques 100. The test apparatus 120 includes a binder clip 130 attached to a dipping stick 140 for a water bath 150, the binder clip 130 configured to hold the slide/plaque 100 or other test article. The water bath 150 is filled with water and an air source 160 infuses air into the water bath 150. The sample slide/plaque 100 is dipped into the water bath 150 just above the air source 160. The results shown in FIGS. 3-9 illustrate observation of air bubble interactions with a fluid facing surface of the slide/plaque 100 within a wet environment as representative of a fluid delivery.

As shown in FIG. 3, the test of a control slide 102 (e.g., uncoated polycarbonate slide) shows air bubbles stuck to the uncoated surface 103 of the uncoated slide 102. In addition, the air bubbles accumulated on the uncoated surface 103 over time. The uncoated control slide 102 provided a contact angle 84 in the range of 65-75 degrees.

A hydrophilic slide 104 is shown in FIG. 4, the hydrophilic slide 104 being the same base polycarbonate as the control slide 102, but coated with a hydrophilic coating having a thickness of 100 nm and providing a contact angle 84 in the range of 38-48 degrees. The hydrophilic slide 104 in FIG. 4 shows no air bubbles stuck to the coated surface 105 of the hydrophilic slide 104. Thus, any air bubbles that contacted the coated surface 105 briefly stuck and popped off, or never stuck to and slid along the coated surface 105.

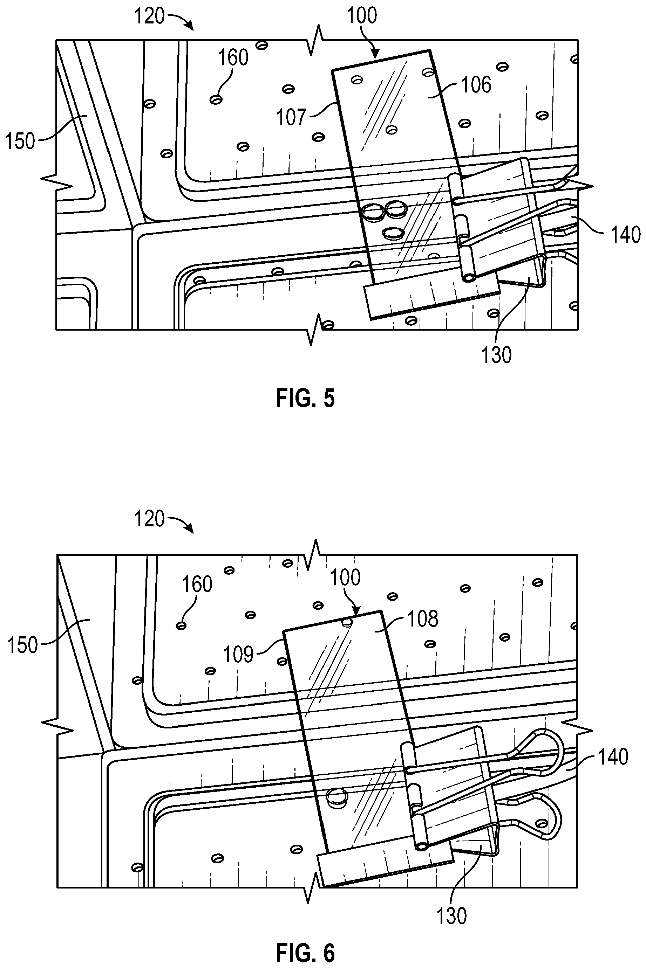

A hydrophobic slide 106 is shown in FIG. 5, the hydrophobic slide 106 being the same base polycarbonate as the control slide 102, but coated with a hydrophobic coating having a thickness of 500 nm and providing a contact angle in the range of 90-94 degrees. The hydrophobic slide 106 in FIG. 5 shows air bubbles stuck to the coated surface 107 of the hydrophobic slide 106. While the uncoated control slide 102 and the hydrophobic slide 106 each have air bubbles stuck to the respective surfaces 103, 107, the size of the air bubbles on the hydrophobic slide 106 are visibly larger.

A super hydrophobic slide 108 is shown in FIG. 6, the super hydrophobic slide 108 being the same base polycarbonate as the control slide 102, but coated with a super hydrophobic coating having a thickness of about 1 µm and providing a contact angle greater than 150 degrees. The super hydrophobic slide 108 in FIG. 6 shows air bubbles stuck to nearly the entire coated surface 109 of the super hydrophobic slide 108. Further, the air bubbles accumulated and aggregated on a large area of the coated surface 109 over time.

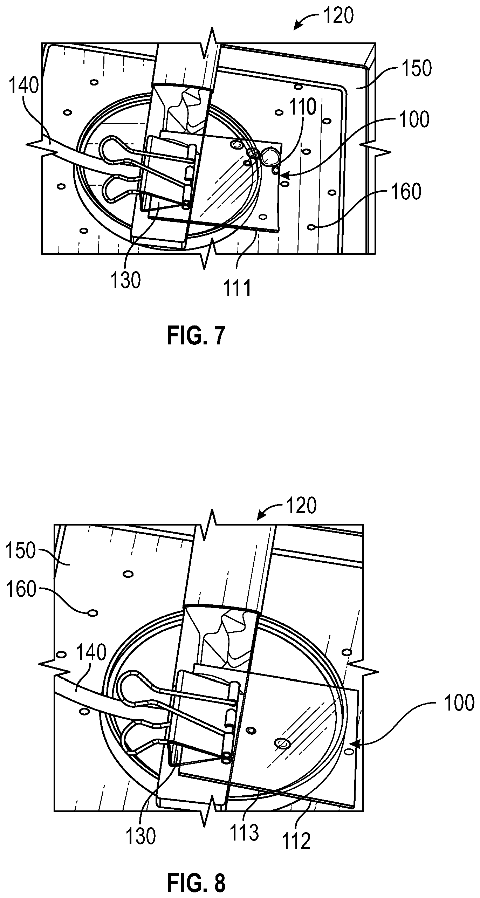

As shown in FIG. 7, a test of another control sample, an uncoated PVC grade plaque 110 representative of IV set pump tubing material, shows air bubbles stuck to the uncoated surface 111 of the uncoated plaque 110. Here again, the air bubbles stuck, accumulated and aggregated to a larger area on the uncoated surface 111 over time.

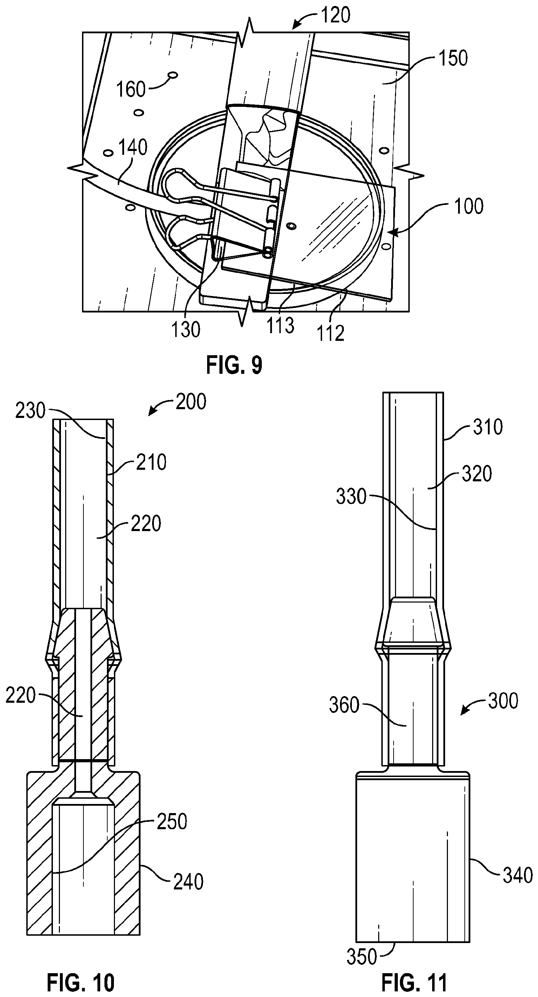

A hydrophilic plaque 112 is shown in FIGS. 8 and 9, the hydrophilic plaque 112 being the same base uncoated PVC grade as the control sample 110 of FIG. 7, but coated with a hydrophilic coating having an indeterminate thickness. The hydrophilic plaque 112 in FIG. 8 shows an air bubble contacting the coated surface 113 of the hydrophilic plaque 112, whereas immediately thereafter in FIG. 9, the air bubble disappeared from the coated surface 113. Thus, air bubbles that contacted the coated surface 113 slid along the coated surface 113 quickly or disappeared by popping away immediately after reaching the coated surface 113.

FIG. 10 shows an example of a hydrophilic tube and infusion component junction 200, according to aspects of the disclosure. The junction 200 includes a tube 210 formed of any suitable material for infusion tubing, the tube 210 having a hydrophilic coating 220 disposed on an internal (e.g., fluid interfacing) surface 230 of the tube 210. The junction 200 also includes an infusion component fluid port 240, the fluid port 240 having a hydrophilic coating 220 disposed on an internal (e.g., fluid interfacing) surface 250 of the fluid port 240. The hydrophilic junction 200 is configured to provide passage of fluid through the tube 210 and the fluid port 240 while minimizing or preventing air bubbles from sticking or accumulating on the internal surface 230 of the tube 210 and the internal surface 250 of the fluid port 240.

FIG. 11 shows an example of a hydrophilic tube and infusion component junction 300, according to aspects of the disclosure. The junction 300 includes a tube 310 formed of a hydrophilic tubing material 320, the tube 310 having an internal (e.g., fluid interfacing) surface 330. The hydrophilic tubing material 320 may be formed by blending any suitable infusion tubing material (e.g., soft PVC) with one or more hydrophilic additives during formation (e.g., extrusion) of the tube 310. The junction 300 also includes an infusion component fluid port 340 formed of a hydrophilic infusion component material 360, the fluid port 340 having an internal (e.g., fluid interfacing) surface 350. The hydrophilic infusion component material 360 may be formed by blending any suitable infusion component material (e.g., PVP) with one or more hydrophilic additives during formation (e.g., molding) of the fluid port 340. The hydrophilic junction 300 is configured to provide passage of fluid through the tube 310 and the fluid port 340 while minimizing or preventing air bubbles from sticking or accumulating on the internal surface 330 of the tube 310 and the internal surface 350 of the fluid port 340.

Additional approaches to create hydrophilic surface properties of fluid interfacing materials are contemplated, according to aspects of the disclosure. For example, simple coating, coating with chemically reactive hydrophilic polymers, formation of impregnated polymer networks, surface grafting of hydrophilic polymers, blending or complexing of hydrophilic polymers, impregnating polymeric materials with an additive in order to change the surface property to impart hydrophilicity, and surface hydrophilic elastomeric latex (SHEL) film formation on elastomeric, rubbery type material.

In one or more embodiments of the disclosure, a hydrophilic infusion tube, comprises a flexible tube configured to connect a first fluid port and a second fluid port, the flexible tube comprising a base material suitable for fluid infusion; and a hydrophilic fluid interfacing surface disposed on an inner surface of the flexible tube, wherein the hydrophilic fluid interfacing surface provides a water wetting contact angle that is less than a water wetting contact angle provided by the base material.

In aspects of the disclosure, the base material comprises one or more of thermoplastic polyurethane (TPU), thermoplastic elastomer (TPE), thermoplastic polyolefin (TPO), soft polyvinyl chloride (PVC), silicone, thermoplastic vulcanizate (TPV) (ethylene propylene diene monomer (EPDM) + polypropylene (PP)), thermoplastic styrenic elastomer (TPS) (styrene-butadiene-styrene (SBS) /styrene-ethylene-butylene-styrene (SEBS) /styrene-isoprene-rubber (SIS) /styrene-ethylene-propylene-styrene (SEPS)) and its blending with polyolefin, and thermoplastic polyester elastomer (TPEE) (polyether ester) rubber). In aspects of the disclosure, the hydrophilic fluid interfacing surface comprises a hydrophilic material coated on the base material. In aspects of the disclosure, the hydrophilic material comprises Guanidinium copolymer. In aspects of the disclosure, the hydrophilic material provides a lower coefficient of friction than a coefficient of friction provided by the base material. In aspects of the disclosure, the hydrophilic material provides a material wettability that is higher than a material wettability provided by the base material.

In aspects of the disclosure, the hydrophilic fluid interfacing surface comprises a hydrophilic material grafted on the base material. In aspects of the disclosure, the hydrophilic fluid interfacing surface comprises a blend of the base material with a hydrophilic additive. In aspects of the disclosure, the hydrophilic additive comprises one or more of polyvinyl pyrrolidone (PVP), polyvinyl alcohol, chitosan (CS), polyamide, polyethylene oxide (PEO), polyethylene glycol (PEG), and polyetheramine. In aspects of the disclosure, the hydrophilic additive comprises Guanidinium copolymer. In aspects of the disclosure, the hydrophilic additive comprises up to 10 percent of the weight of the base material and hydrophilic additive combined. In aspects of the disclosure, the hydrophilic additive comprises up to 20 percent of the weight of the base material and hydrophilic additive combined.

In one or more embodiments of the disclosure, a hydrophilic infusion component comprises a housing comprising a first fluid port; a second fluid port; a base material suitable for fluid infusion; and a fluid pathway disposed between the first fluid port and the second fluid port. The hydrophilic infusion component also comprises a hydrophilic fluid interfacing surface disposed on an inner surface of the fluid pathway, wherein the hydrophilic fluid interfacing surface provides a water wetting contact angle that is less than a water wetting contact angle provided by the base material.

In aspects of the disclosure, the base material comprises one or more of thermoplastic polyurethane (TPU), thermoplastic elastomer (TPE), thermoplastic polyolefin (TPO), soft polyvinyl chloride (PVC), silicone, thermoplastic vulcanizate (TPV) (ethylene propylene diene monomer (EPDM) + polypropylene (PP)), thermoplastic styrenic elastomer (TPS) (styrene-butadiene-styrene (SBS) /styrene-ethylene-butylene-styrene (SEBS) /styrene-isoprene-rubber (SIS) /styrene-ethylene-propylene-styrene (SEPS)) and its blending with polyolefin, and thermoplastic polyester elastomer (TPEE) (polyether ester) rubber).

In aspects of the disclosure, the hydrophilic fluid interfacing surface comprises a hydrophilic material coated on the base material. In aspects of the disclosure, the hydrophilic fluid interfacing surface comprises a hydrophilic material grafted on the base material. In aspects of the disclosure, the hydrophilic fluid interfacing surface comprises a blend of the base material with a hydrophilic additive. In aspects of the disclosure, the hydrophilic additive comprises one or more of polyvinyl pyrrolidone (PVP), polyvinyl alcohol, chitosan (CS), polyamide, polyethylene oxide (PEO), polyethylene glycol (PEG), polyetheramine, and Guanidinium copolymer.

In one or more embodiments of the disclosure, an infusion set comprises an infusion component and a hydrophilic infusion tube. The hydrophilic infusion tube comprises a flexible tube configured to connect a fluid port of the infusion component to a different fluid port, the flexible tube comprising a base material suitable for fluid infusion; and a hydrophilic fluid interfacing surface disposed on an inner surface of the flexible tube, wherein the hydrophilic fluid interfacing surface provides a water wetting contact angle that is less than a water wetting contact angle provided by the base material, and wherein the flexible tube is configured to provide fluid passage without an accumulation of air bubbles on the inner surface of the flexible tube.

In aspects of the disclosure, the infusion component is a hydrophilic infusion component comprising: a housing comprising a first fluid port; a second fluid port; a base material suitable for fluid infusion; and a fluid pathway disposed between the first fluid port and the second fluid port. The hydrophilic infusion component also comprises a hydrophilic fluid interfacing surface disposed on an inner surface of the fluid pathway, wherein the hydrophilic fluid interfacing surface provides a water wetting contact angle that is less than a water wetting contact angle provided by the base material, and wherein the fluid pathway is configured to provide fluid passage without an accumulation of air bubbles on the inner surface of the fluid pathway.

It is understood that any specific order or hierarchy of blocks in the methods of processes disclosed is an illustration of example approaches. Based upon design or implementation preferences, it is understood that the specific order or hierarchy of blocks in the processes may be rearranged, or that all illustrated blocks be performed. In some implementations, any of the blocks may be performed simultaneously.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

It is understood that the specific order or hierarchy of steps, operations or processes disclosed is an illustration of exemplary approaches. Based upon design preferences, it is understood that the specific order or hierarchy of steps, operations or processes may be rearranged. Some of the steps, operations or processes may be performed simultaneously. Some or all of the steps, operations, or processes may be performed automatically, without the intervention of a user. The accompanying method claims, if any, present elements of the various steps, operations or processes in a sample order, and are not meant to be limited to the specific order or hierarchy presented.

Moreover, nothing disclosed herein is intended to be dedicated to the public regardless of whether such disclosure is explicitly recited in the claims. Furthermore, to the extent that the term "include," "have," or the like is used, such term is intended to be inclusive in a manner similar to the term "comprise" as "comprise" is interpreted when employed as a transitional word in a claim.

The Title, Background, Summary, Brief Description of the Drawings and Abstract of the disclosure are hereby incorporated into the disclosure and are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The following claims are hereby incorporated into the Detailed Description, with each claim standing on its own as a separately claimed subject matter.

## Claims

1. A hydrophilic infusion tube (310), comprising:
a flexible tube configured to connect a first fluid port and a second fluid port, the flexible tube comprising:
a base material suitable for fluid infusion; and
a hydrophilic fluid interfacing surface (330) forming an inner surface of the flexible tube,
wherein the hydrophilic fluid interfacing surface (330) provides a water wetting contact angle that is less than a water wetting contact angle provided by the base material,
wherein the hydrophilic fluid interfacing surface (330) comprises a blend of the base material with a hydrophilic additive during formation of the flexible tube.

2. The hydrophilic infusion tube (310) of claim 1, wherein the base material comprises one or more of thermoplastic polyurethane (TPU), thermoplastic elastomer (TPE), thermoplastic polyolefin (TPO), soft polyvinyl chloride (PVC), silicone, thermoplastic vulcanizate (TPV) (ethylene propylene diene monomer (EPDM) + polypropylene (PP)), thermoplastic styrenic elastomer (TPS) (styrene-butadiene-styrene (SBS) /styrene-ethylene-butylene-styrene (SEBS) /styrene-isoprene-rubber (SIS) /styrene-ethylene-propylene-styrene (SEPS)) and its blending with polyolefin, and thermoplastic polyester elastomer (TPEE) (polyether ester) rubber).

3. The hydrophilic infusion tube (310) of claim 1, wherein the hydrophilic fluid interfacing surface (330) provides a lower coefficient of friction than a coefficient of friction provided by the base material.

4. The hydrophilic infusion tube (310) of claim 3, wherein the hydrophilic fluid interfacing surface (330) provides a material wettability that is higher than a material wettability provided by the base material.

5. The hydrophilic infusion tube (310) of claim 1, wherein the hydrophilic additive comprises one or more of polyvinyl pyrrolidone (PVP), polyvinyl alcohol, chitosan (CS), polyamide, polyethylene oxide (PEO), polyethylene glycol (PEG), and polyetheramine.

6. The hydrophilic infusion tube (310) of claim 1, wherein the hydrophilic additive comprises Guanidinium copolymer.

7. The hydrophilic infusion tube (310) of claim 1, wherein the hydrophilic additive comprises up to 10 percent of the weight of the base material and hydrophilic additive combined.

8. The hydrophilic infusion tube (310) of claim 1, wherein the hydrophilic additive comprises up to 20 percent of the weight of the base material and hydrophilic additive combined.

9. An infusion set, comprising:
an infusion component; and
the hydrophilic infusion tube (310) of claim 1,
wherein the flexible tube is configured to provide fluid passage without an accumulation of air bubbles on the inner surface of the flexible tube.

10. The infusion set of claim 9, wherein the infusion component (340) is a hydrophilic infusion component (340) comprising:
a housing comprising:
a first fluid port;
a second fluid port;
a base material suitable for fluid infusion; and
a fluid pathway disposed between the first fluid port and the second fluid port; and
a hydrophilic fluid interfacing surface (350) disposed on an inner surface of the fluid pathway,
wherein the hydrophilic fluid interfacing surface (350) provides a water wetting contact angle that is less than a water wetting contact angle provided by the base material,
wherein the fluid pathway is configured to provide fluid passage without an accumulation of air bubbles on the inner surface of the fluid pathway.

11. The infusion set of claim 10, wherein the base material of the hydrophilic infusion component (340) comprises one or more of thermoplastic polyurethane (TPU), thermoplastic elastomer (TPE), thermoplastic polyolefin (TPO), soft polyvinyl chloride (PVC), silicone, thermoplastic vulcanizate (TPV) (ethylene propylene diene monomer (EPDM) + polypropylene (PP)), thermoplastic styrenic elastomer (TPS) (styrene-butadiene-styrene (SBS) /styrene-ethylene-butylene-styrene (SEBS) /styrene-isoprene-rubber (SIS) /styrene-ethylene-propylene-styrene (SEPS)) and its blending with polyolefin, and thermoplastic polyester elastomer (TPEE) (polyether ester) rubber).

12. The infusion set of claim 10, wherein the hydrophilic fluid interfacing surface of the hydrophilic infusion component (340) comprises a hydrophilic material coated on the base material.

13. The infusion set of claim 10, wherein the hydrophilic fluid interfacing surface of the hydrophilic infusion component (340) comprises a hydrophilic material grafted on the base material.

14. The infusion set of claim 10, wherein the hydrophilic fluid interfacing surface of the hydrophilic infusion component (340) comprises a blend of the base material with a hydrophilic additive.

15. The infusion set of claim 14, wherein the hydrophilic additive comprises one or more of polyvinyl pyrrolidone (PVP), polyvinyl alcohol, chitosan (CS), polyamide, polyethylene oxide (PEO), polyethylene glycol (PEG), polyetheramine, and Guanidinium copolymer.

## Patentansprüche

1. Hydrophiler Infusionsschlauch (310), umfassend:
einen flexiblen Schlauch, der dazu konfiguriert ist, einen ersten Fluidanschluss und einen zweiten Fluidanschluss zu verbinden, wobei der flexible Schlauch umfasst:
ein für die Fluidinfusion geeignetes Basismaterial; und
eine hydrophile Fluidkontaktfläche (330), die eine Innenfläche des flexiblen Schlauchs bildet,
wobei die hydrophile Fluidkontaktfläche (330) einen Wasserbenetzungswinkel bereitstellt, der kleiner ist als ein vom Basismaterial bereitgestellter Wasserbenetzungswinkel,
wobei die hydrophile Fluidkontaktfläche (330) eine Mischung des Basismaterials mit einem hydrophilen Additiv während der Bildung des flexiblen Schlauchs umfasst.

2. Hydrophiler Infusionsschlauch (310) nach Anspruch 1, wobei das Basismaterial eines oder mehrere der folgenden Materialien umfasst: thermoplastisches Polyurethan (TPU), thermoplastisches Elastomer (TPE), thermoplastisches Polyolefin (TPO), weiches Polyvinylchlorid (PVC), Silikon, thermoplastisches Vulkanisat (TPV) (Ethylen-Propylen-Dien-Monomer (EPDM) + Polypropylen (PP)), thermoplastisches Styrol-Elastomer (TPS) (Styrol-Butadien-Styrol (SBS) / Styrol-Ethylen-Butylen-Styrol (SEBS) / Styrol-Isopren-Kautschuk (SIS) / Styrol-Ethylen-Propylen-Styrol (SEPS)) und dessen Mischung mit Polyolefin, und thermoplastisches Polyesterelastomer (TPEE) (Polyetherester-Kautschuk).

3. Hydrophiler Infusionsschlauch (310) nach Anspruch 1, wobei die hydrophile Fluidkontaktfläche (330) einen niedrigeren Reibungskoeffizienten bereitstellt als ein vom Basismaterial bereitgestellter Reibungskoeffizient.

4. Hydrophiler Infusionsschlauch (310) nach Anspruch 3, wobei die hydrophile Fluidkontaktfläche (330) eine Materialbenetzbarkeit bereitstellt, die höher ist als eine vom Basismaterial bereitgestellte Materialbenetzbarkeit.

5. Hydrophiler Infusionsschlauch (310) nach Anspruch 1, wobei das hydrophile Additiv eines oder mehrere der folgenden umfasst: Polyvinylpyrrolidon (PVP), Polyvinylalkohol, Chitosan (CS), Polyamid, Polyethylenoxid (PEO), Polyethylenglykol (PEG) und Polyetheramin.

6. Hydrophiler Infusionsschlauch (310) nach Anspruch 1, wobei das hydrophile Additiv Guanidinium-Copolymer umfasst.

7. Hydrophiler Infusionsschlauch (310) nach Anspruch 1, wobei das hydrophile Additiv bis zu 10 Prozent des Gewichts des Basismaterials und des hydrophilen Additivs zusammen umfasst.

8. Hydrophiler Infusionsschlauch (310) nach Anspruch 1, wobei das hydrophile Additiv bis zu 20 Prozent des Gewichts des Basismaterials und des hydrophilen Additivs zusammen umfasst.

9. Infusionsset, umfassend:
eine Infusionskomponente; und
den hydrophilen Infusionsschlauch (310) nach Anspruch 1,
wobei der flexible Schlauch dazu konfiguriert ist, einen Fluiddurchgang ohne eine Ansammlung von Luftblasen an der Innenfläche des flexiblen Schlauchs bereitzustellen.

10. Infusionsset nach Anspruch 9, wobei die Infusionskomponente (340) eine hydrophile Infusionskomponente (340) ist, die umfasst:
ein Gehäuse, das umfasst:
einen ersten Fluidanschluss;
einen zweiten Fluidanschluss;
ein für die Fluidinfusion geeignetes Basismaterial; und
einen Fluidweg, der zwischen dem ersten Fluidanschluss und dem zweiten Fluidanschluss angeordnet ist; und
eine hydrophile Fluidkontaktfläche (350), die an einer Innenfläche des Fluidwegs angeordnet ist,
wobei die hydrophile Fluidkontaktfläche (350) einen Wasserbenetzungswinkel bereitstellt, der kleiner ist als ein vom Basismaterial bereitgestellter Wasserbenetzungswinkel,
wobei der Fluidweg dazu konfiguriert ist, einen Fluiddurchgang ohne eine Ansammlung von Luftblasen an der Innenfläche des Fluidwegs bereitzustellen.

11. Infusionsset nach Anspruch 10, wobei das Basismaterial der hydrophilen Infusionskomponente (340) eines oder mehrere der folgenden Materialien umfasst: thermoplastisches Polyurethan (TPU), thermoplastisches Elastomer (TPE), thermoplastisches Polyolefin (TPO), weiches Polyvinylchlorid (PVC), Silikon, thermoplastisches Vulkanisat (TPV) (Ethylen-Propylen-Dien-Monomer (EPDM) + Polypropylen (PP)), thermoplastisches Styrol-Elastomer (TPS) (Styrol-Butadien-Styrol (SBS) / Styrol-Ethylen-Butylen-Styrol (SEBS) / Styrol-Isopren-Kautschuk (SIS) / Styrol-Ethylen-Propylen-Styrol (SEPS)) und dessen Mischung mit Polyolefin, und thermoplastisches Polyesterelastomer (TPEE) (Polyetherester-Kautschuk).

12. Infusionsset nach Anspruch 10, wobei die hydrophile Fluidkontaktfläche der hydrophilen Infusionskomponente (340) ein auf das Basismaterial aufgebrachtes hydrophiles Material umfasst.

13. Infusionsset nach Anspruch 10, wobei die hydrophile Fluidkontaktfläche der hydrophilen Infusionskomponente (340) ein auf das Basismaterial aufgepfropftes hydrophiles Material umfasst.

14. Infusionsset nach Anspruch 10, wobei die hydrophile Fluidkontaktfläche der hydrophilen Infusionskomponente (340) eine Mischung des Basismaterials mit einem hydrophilen Additiv umfasst.

15. Infusionsset nach Anspruch 14, wobei das hydrophile Additiv eines oder mehrere der folgenden umfasst: Polyvinylpyrrolidon (PVP), Polyvinylalkohol, Chitosan (CS), Polyamid, Polyethylenoxid (PEO), Polyethylenglykol (PEG), Polyetheramin und Guanidinium-Copolymer.

## Revendications

1. Tube de perfusion hydrophile (310), comprenant :
un tube flexible configuré pour relier un premier orifice de fluide et un deuxième orifice de fluide, le tube flexible comprenant :
un matériau de base adapté à la perfusion de fluide ; et
une surface d'interface avec le fluide hydrophile (330) formant une surface interne du tube flexible,
dans lequel la surface d'interface avec le fluide hydrophile (330) fournit un angle de contact mouillant avec l'eau qui est inférieur à un angle de contact mouillant avec l'eau fourni par le matériau de base,
dans lequel la surface d'interface avec le fluide hydrophile (330) comprend un mélange du matériau de base avec un additif hydrophile pendant la formation du tube flexible.

2. Tube de perfusion hydrophile (310) selon la revendication 1, dans lequel le matériau de base comprend un ou plusieurs des matériaux suivants : polyuréthane thermoplastique (TPU), élastomère thermoplastique (TPE), polyoléfine thermoplastique (TPO), chlorure de polyvinyle souple (PVC), silicone, vulcanisat thermoplastique (TPV) (éthylène-propylène-diène monomère (EPDM) + polypropylène (PP)), élastomère styrénique thermoplastique (TPS) (styrène-butadiène-styrène (SBS) / styrène-éthylène-butylène-styrène (SEBS) / styrène-isoprène-caoutchouc (SIS) / styrène-éthylène-propylène-styrène (SEPS)) et son mélange avec de la polyoléfine, et élastomère thermoplastique polyester (TPEE) (caoutchouc polyéther ester).

3. Tube de perfusion hydrophile (310) selon la revendication 1, dans lequel la surface d'interface avec le fluide hydrophile (330) fournit un coefficient de frottement inférieur à un coefficient de frottement fourni par le matériau de base.

4. Tube de perfusion hydrophile (310) selon la revendication 3, dans lequel la surface d'interface avec le fluide hydrophile (330) fournit une mouillabilité du matériau qui est supérieure à une mouillabilité du matériau fournie par le matériau de base.

5. Tube de perfusion hydrophile (310) selon la revendication 1, dans lequel l'additif hydrophile comprend un ou plusieurs des composés suivants : polyvinylpyrrolidone (PVP), alcool polyvinylique, chitosane (CS), polyamide, oxyde de polyéthylène (PEO), polyéthylène glycol (PEG) et polyétheramine.

6. Tube de perfusion hydrophile (310) selon la revendication 1, dans lequel l'additif hydrophile comprend un copolymère de guanidinium.

7. Tube de perfusion hydrophile (310) selon la revendication 1, dans lequel l'additif hydrophile comprend jusqu'à 10 pour cent du poids du matériau de base et de l'additif hydrophile combinés.

8. Tube de perfusion hydrophile (310) selon la revendication 1, dans lequel l'additif hydrophile comprend jusqu'à 20 pour cent du poids du matériau de base et de l'additif hydrophile combinés.

9. Ensemble de perfusion, comprenant :
un composant de perfusion ; et
le tube de perfusion hydrophile (310) selon la revendication 1,
dans lequel le tube flexible est configuré pour fournir un passage de fluide sans accumulation de bulles d'air sur la surface interne du tube flexible.

10. Ensemble de perfusion selon la revendication 9, dans lequel le composant de perfusion (340) est un composant de perfusion hydrophile (340) comprenant :
un boîtier comprenant :
un premier orifice de fluide ;
un deuxième orifice de fluide ;
un matériau de base adapté à la perfusion de fluide ; et
un passage de fluide disposé entre le premier orifice de fluide et le deuxième orifice de fluide ; et
une surface d'interface avec le fluide hydrophile (350) disposée sur une surface interne du passage de fluide,
dans lequel la surface d'interface avec le fluide hydrophile (350) fournit un angle de contact mouillant avec l'eau qui est inférieur à un angle de contact mouillant avec l'eau fourni par le matériau de base,
dans lequel le passage de fluide est configuré pour fournir un passage de fluide sans accumulation de bulles d'air sur la surface interne du passage de fluide.

11. Ensemble de perfusion selon la revendication 10, dans lequel le matériau de base du composant de perfusion hydrophile (340) comprend un ou plusieurs des matériaux suivants : polyuréthane thermoplastique (TPU), élastomère thermoplastique (TPE), polyoléfine thermoplastique (TPO), chlorure de polyvinyle souple (PVC), silicone, vulcanisat thermoplastique (TPV) (éthylène-propylène-diène monomère (EPDM) + polypropylène (PP)), élastomère styrénique thermoplastique (TPS) (styrène-butadiène-styrène (SBS) / styrène-éthylène-butylène-styrène (SEBS) / styrène-isoprène-caoutchouc (SIS) / styrène-éthylène-propylène-styrène (SEPS)) et son mélange avec de la polyoléfine, et élastomère thermoplastique polyester (TPEE) (caoutchouc polyéther ester).

12. Ensemble de perfusion selon la revendication 10, dans lequel la surface d'interface avec le fluide hydrophile du composant de perfusion hydrophile (340) comprend un matériau hydrophile revêtu sur le matériau de base.

13. Ensemble de perfusion selon la revendication 10, dans lequel la surface d'interface avec le fluide hydrophile du composant de perfusion hydrophile (340) comprend un matériau hydrophile greffé sur le matériau de base.

14. Ensemble de perfusion selon la revendication 10, dans lequel la surface d'interface avec le fluide hydrophile du composant de perfusion hydrophile (340) comprend un mélange du matériau de base avec un additif hydrophile.

15. Ensemble de perfusion selon la revendication 14, dans lequel l'additif hydrophile comprend un ou plusieurs des composés suivants : polyvinylpyrrolidone (PVP), alcool polyvinylique, chitosane (CS), polyamide, oxyde de polyéthylène (PEO), polyéthylène glycol (PEG), polyétheramine et copolymère de guanidinium.
